# EUROPÄISCHE PATENTSCHRIFT

(11) **EP 2 094 080 B1**
(45) Veröffentlichungstag und Bekanntmachung des Hinweises auf die Patenterteilung: **07.06.2017**
(21) Anmeldenummer: 07822106.6
(22) Anmeldetag: 31.10.2007
(51) Int. Cl.: A01N 25/30

(54) **VERWENDUNG VON BLOCKCOPOLYMEREN AUF BASIS VON VINYLLACTAMEN UND VINYLACETAT ALS SOLUBILISATOREN**
UTILISATION OF BLOCK COPOLYMERS BASED ON VINYL LACTAMES AND VINYL ACETATE AS SOLUBILISERS
UTILISATION DE COPOLYMÈRES BLOCS À BASE DE LACTAME VINYLIQUE ET D'ACÉTATE VINYLIQUE EN TANT QUE SOLUBILISATEURS

(30) Priorität: 13.11.2006 EP 06123959; 05.12.2006 EP 06125423
(43) Veröffentlichungstag der Anmeldung: 02.09.2009
(73) Patentinhaber: BASF SE, 67056 Ludwigshafen am Rhein (DE)
(72) Erfinder: DIECKMANN, Yvonne, 67454 Hassloch (DE); MERTOGLU, Murat, 67059 Ludwigshafen (DE); DOBRAWA, Rainer, 68167 Mannheim (DE); CSIHONY, Szilard, 69469 Weinheim (DE); DIELEMAN, Cedric, F-67630 Scheibenhard (FR); KNIERIEM, Torsten, 68305 Mannheim (DE); KOLTZENBURG, Sebastian, 67125 Dannstadt-Schauernheim (DE); TÜRK, Holger, 68161 Mannheim (DE); TROPPMANN, Ulrike, 68163 Mannheim (DE); JUNG, Christian Michael, 67067 Ludwigshafen (DE)
(74) Vertreter: BASF IP Association
(86) Internationale Anmeldenummer: PCT/EP2007/061759
(87) Internationale Veröffentlichungsnummer: WO 2008/058848

(56) Entgegenhaltungen:
- EP-A- 1 027 886
- EP-A1- 0 781 550
- EP-A2- 1 269 994
- WO-A-00/53164
- WO-A2-2004/019901
- WO-A2-2004/035013
- DE-B- 1 245 542
- US-A- 5 502 136

## Beschreibung

Die vorliegende Erfindung betrifft die Verwendung von Blockcopolymeren auf Basis von Vinyllactamen und Vinylacetat als Kristallisationsinhibitoren für in Wasser schwerlösliche Wirkstoffe, insbesondere von Pestiziden (agrochemischen Wirkstoffen).

Bei der Herstellung homogener Zubereitungen insbesondere von biologisch aktiven Substanzen hat die Solubilisierung von hydrophoben, also in Wasser schwerlöslichen Stoffen, eine sehr große praktische Bedeutung erlangt.

Unter Solubilisierung ist das Löslichmachen von in einem bestimmtem Lösungsmittel, insbesondere Wasser, schwer- oder unlöslichen Substanzen durch grenzflächenaktive Verbindungen, die Solubilisatoren, zu verstehen. Solche Solubilisatoren sind in der Lage, schlecht wasserlösliche oder wasserunlösliche Stoffe in klare, höchstens opaleszierende wässrige Lösungen zu überführen, ohne dass hierbei die chemische Struktur dieser Stoffe eine Veränderung erfährt (Vgl. Römpp Chemie Lexikon, 9. Auflage, Bd.5. S. 4203, Thieme Verlag, Stuttgart, 1992).

Die hergestellten Solubilisate sind dadurch gekennzeichnet, dass der schlecht wasserlösliche oder wasserunlösliche Stoff in den Molekülassoziaten der oberflächenaktiven Verbindungen, die sich in wässriger Lösung bilden, wie beispielsweise hydrophobe Domänen oder Mizellen, kolloidal gelöst vorliegt. Die resultierenden Lösungen sind stabile oder metastabile einphasige Systeme, die optisch klar bis opaleszent erscheinen.

Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich Tenside wie ethoxyliertes Ricinusöl oder ethoxyliertes hydriertes Ricinusöl, ethoxylierte Sorbitanfettsäureester oder ethoxylierte Hydroxystearinsäure eingesetzt.

Die oben beschriebenen, bisher eingesetzten Solubilisatoren zeigen jedoch eine Reihe anwendungstechnischer Nachteile.

Die bekannten Solubilisatoren besitzen für einige schwerlösliche Arzneistoffe wie z.B. Clotrimazol nur eine geringe lösungsvermittelnde Wirkung.

In der EP-A 876 819 ist die Verwendung von Copolymeren aus mindestens 60 Gew.-% N-Vinylpyrrolidon und Amiden oder Estern mit langkettigen Alkylgruppen beschrieben.

In der EP-A 948 957 ist die Verwendung von Copolymerisaten aus monoethylenisch ungesättigten Carbonsäuren wie beispielsweise Acrylsäure und hydrophob modifizierten Comonomeren wie beispielsweise N-Alkyl- oder N,N-Dialkyl- Amiden ungesättigter Carbonsäuren mit Cs-Cso-Alkylresten beschrieben.

Aus der DE-A 199 350 63 sind Polyalkylenoxid-haltige Pfropfpolymere auf Basis von Vinyllactamen und Vinylacetat sowie deren Verwendung als Gashydratinhibitoren bekannt.

Aus der EP-A 953 347 ist die Verwendung von Polyalkylenoxid-haltigen Pfropfpolymerisaten als Solubilisatoren bekannt. Die dort beschriebenen Pfropfpolymerisate aus Vinylacetat und Polyalkylenoxiden stellen häufig keine Pulver sondern zähklebrige Flüssigkeiten dar, was anwendungstechnisch von Nachteil ist.

Aus EP 0781 550 ist die Verwendung von statistischen Copolymeren von Vinylpyrrolidon und Vinylacetat als Bioadhäsionsmittel in der Pharmazie bekannt.

Aus DE 1245542 ist die Verwendung von statistischen Mischpolymeren von Polyvinylpyrolidon mit Vinylacetat als Lösungsmittel für Peptidantibiotika bekannt. Als Blockmischpolymer wird das Blockmischpolymer von Polyoxyethylen und Polyoxypropylen genannt.

Zahlreiche Typen von Blockpolymeren werden durch ionische Polymerisation hergestellt. Allerdings eignet sich dieses Verfahren nicht für alle Monomeren. Einer Polymerisation über radikalisch iniitierte Verfahren ist eine große Anzahl von Monomeren zugänglich, jedoch ist eine normale radikalische Polymerisation nicht zur Herstellung von Blockcopolymeren anwendbar.

Daher wurde das Verfahren der kontrollierten radikalischen Polymerisation, die auch als "lebende" Polymerisation bezeichnet wird, entwickelt.

Eine Variante der lebenden radikalischen Polymerisation ist die sogenannte "RAFT"-Methode (RAFT: Reversible Addition- Fragmentation chain Transfer). Dabei kommen als Kettenübertragungsmittel, die auch als RAFT-Reagenzien bezeichnet werden bestimmte Schwefelverbindungen in Betracht, beispielsweise Dithiocarbamate oder Xanthate.

In dieser EP-B 991 683 ist die Herstellung von Blockcopolymeren aus Polyvinylacetat und Polyalkylacrylatblöcken über lebende Polymerisation mit Xanthaten beschrieben.

Aus der WO 98/01478 ist die Herstellung von Blockpolymerisaten aus Polyalkylacrylaten und Polystyrolen über lebende Polymerisation mit Thiocarbonylthioverbindungen als Kettenübertragungsmittel bekannt.

In der EP-A 1510533 ist die Herstellung von Polyvinyllactamblöcke enthaltende Blockcopolymeren durch lebende Polymerisation beschrieben. Der Polyvinyllactamblock kann dabei auch ein Copolymer aus Vinyllactam und bis zu 45 Gew.-% Vinylacetat sein. Als Co-Blöcke werden Polykohlenwasserstoffe oder Poly(meth)acrylate beschrieben.

Aus P. Bilalis et al., Journal of Polymer science : Part A, Vol. 44, 659-665 (2006) ist die Herstellung von Polyvinylpyrrolidon-Blockcopolymeren mittels RAFT-Polymerisation bekannt.

T.L. Uyen Nguyen et al. beschreiben in Journal of Polymer Science: PartA, Vol. 44, 4372-4383 (2006) die Herstellung von Blockcopolymeren aus Polyvinylpyrrolidon- und Polyvinylacetat-Blöcken durch kontrollierte Polymerisation sowie die Verwendung solcher Blockcopolymere als Stabilisierungsmittel bei der Suspensionspolymerisation spezieller vernetzter Polymer-Mikrosphären.

Eine weitere wünschenswerte Anforderung an Solubilisatoren ist die Fähigkeit, mit schwerlöslichen Substanzen sogenannte "feste Lösungen" auszubilden. Der Begriff "feste Lösung" bezeichnet einen Zustand, in dem eine Substanz mikrodispers oder im Idealfall molekulardispers in einer festen Matrix, beispielsweise einer Polymermatrix, verteilt ist. Solche festen Lösungen führen zum Beispiel bei Verwendung in festen pharmazeutischen Darreichungsformen eines schwerlöslichen Wirkstoffs zu einer verbesserten Freisetzung des Wirkstoffs. Eine wichtige Anforderung an solche feste Lösungen ist, dass sie auch bei Lagerung über längere Zeit stabil sind, d.h., dass der Wirkstoff nicht auskristallisiert. Weiterhin ist auch die Kapazität der festen Lösung, mit anderen Worten die Fähigkeit der Ausbildung von stabilen festen Lösungen mit möglichst hohen Wirkstoffgehalten, von Bedeutung.

Als feste Lösung wird hier ein Zustand bezeichnet, bei dem der Wirkstoff molekulardispers verteilt in einer Matrix aus Hilfsstoffen vorliegt. In diesem Zustand sind röntgendiffraktometrisch keine kristallinen Anteile des Wirkstoffs mehr festzustellen. Da die Nachweisgrenze für kristalline Anteile bei der Röntgendiffraktometrie bei 3 Gew.-% liegt, bedeutet der Ausdruck "keine kristallinen Anteile",dass weniger als 3 Gew.- % kristalline Anteile vorhanden sind. Der Zustand der molekulardispersen Verteilung kann mit Hilfe der Methode der Differential Scanning Calorimetry (DSC) ermittelt werden. Bei einer molekulardispersen Verteilung ist im Bereich des Schmelzpunkts des Wirkstoffs kein Schmelzpeak mehr zu beobachten. Die Nachweisgrenze dieser Methode liegt bei 1 Gew.- %. In der WO 05/046328 werden Beispielen von feste Lösungen gegeben.

Bei der Ausbildung von festen Lösungen spielt neben der grundsätzlichen Fähigkeit der Solublisatoren zur Bildung von festen Lösungen auch die Hygroskopizität der Solubilisatoren eine bedeutende Rolle. Solubilisatoren, die aus der Umgebungsluft zuviel Wasser aufnehmen, führen zu einem Zerfliessen der festen Lösung und der unerwünschten Kristallisation der Wirkstoffe. Auch bei der Verarbeitung zu Darreichungsformen bzw. zu festen agrochemischen Zubereitungen kann eine zu große Hygroskopizität Probleme bereiten.

Insbesondere bei agrochemischen Zubereitungen kann dies zu Problemen bei der Lagerung durch sog. Verklebung führen.

Die bisher bekannten polymeren Solubilisatoren weisen die Nachteile auf, dass sie keine stabilen festen Lösungen ausbilden. Ausserdem lassen sie noch Raum für Verbesserungen, was die Solubilisierung in wässrigen Systemen betrifft. Auch hinsichtlich der Verarbeitbarkeit weisen einige der bekannten Solubilisatoren aufgrund ihrer Neigung zu Klebrigkeit Nachteile auf, da sie keine ausreichend fließfähigen Pulver darstellen.

Des weiteren ist es insbesondere bei agrochemischen Zubereitungen von Wichtigkeit, eine hohe Lagerstabilität der Zubereitung durch Wahl geeigneter Solubilisatoren zu erzielen (z.B. durch Vermeidung von Kristallisationsbildung) und / oder durch Wahl eines geeigneten Solubilisators die Bioverfügbarkeit des Pestizides zu erhöhen und/oder eine möglichst geringe Phytotoxizität aufweisen. Es besteht hier ein stetiger Bedarf, geeignete Solubilisatoren zu finden.

Bei der Formulierung von in Wasser schwerlöslichen Wirkstoffen besteht das Problem, dass der schwerlösliche Wirkstoff in der wässerigen Formulierung zur Kristallisation während der Lagerung neigt. Dieses Problem ist bei schwer löslichen Wirkstoffen naturgemäß eng verbundenen Problem der mangelnden Solubilisierung.

Es bestand daher die Aufgabe, verbesserte Solubilisatoren für agrochemische Anwendungen bereitzustellen, die die geschilderten Nachteile nicht aufweisen. Darüber hinaus ist es eine Aufgabe der vorliegenden Erfindung, Solubilisatoren insbesondere für schwerlösliche Pestizide bereitzustellen, welche eine hohe Lagerstabilität der agrochemischen Zubereitungen ermöglichen und / oder die Bioverfügbarkeit des Pestizides erhöhen und/oder eine möglichst geringe Phytotoxizität aufweisen.

Aufgabe der vorliegenden Erfindung war es, wie bereits ausgeführt, verbesserte Copolymere als Kristallisationsinhibitoren zur Verfügung zu stellen.

Demgemäß wurde die Verwendung von Blockcopolymeren, bestehend aus wenigstens einem Polyvinyllactam-Block und wenigstens einem Polyvinylacetat-Block, als Solubilisatoren für in Wasser schwerlösliche Wirkstoffe gefunden.Demgemäß wurde außerdem die Verwendung von Blockcopolymeren, bestehend aus wenigstens einem Polyvinyllactam-Block und wenigstens einem Polyvinylacetat-Block, als Kristallisationsinhibitoren für in Wasser schwerlösliche Wirkstoffe, bevorzugt Pestizide, gefunden. Außerdem wurde gefunden, dass die erfindungsgemäßen Blockcopolymere die oben genannten Probleme der Kristallisationsinhibierung lösen können.

Als Wirkstoffe im Sinne der vorliegenden Erfindung werden biologisch aktive Substanzen für agrochemische Anwendungen (auch Pestizide oder agrochemische Wirkstoffe genannt) verstanden.

Insbesondere sind diese Blockcopolymere als Kristallisationsinhibitoren für in Wasser schwerlösliche Pestzide geeignet.

Die Blockcopolymere können vom AB-, ABA- oder BAB-Typ sein.

Als Polyvinyllactam kommen N-vinylpyrrolidon, N-Vinylpiperidon oder N-Vinylcaprolactam in Betracht, bevorzugt Polyvinylpyrrolidon.

Das Molverhältnis von Polyvinyllactam zu Polyvinylacetat (PVAc) kann 10 zu 90 bis 90 zu 10 betragen, bevorzugt 30 zu 70 bis 70 zu 30, besonders bevorzugt 60 : 40 bis 40 : 60.

Im folgenden wird der Polyvinyllactam- Block auch als A-Block und der Polyvinylacetat-Block als B-Block bezeichnet.

Grundsätzlich können die Blockcopolymere durch jedes hierfür geeignete Verfahren hergestellt werden.

So kann zum Beispiel eine Kupplung der PVP- und der PVAc-Böcke über Diisocyanate erfolgen.

Um die Ankupplung des Polyvinylacetat-Blocks an den Polyvinyllactam-Block zu ermöglichen, sind die Polymerblöcke am Kettenanfang und/oder am Kettenende mit Hydroxyl-Gruppen funktionalisiert. Die OH-Funktionalisierung kann entweder über den Radikalstarter oder über einen Regler erzielt werden. Die Funktionalisierung über den Radikalstarter erfolgt am Kettenanfang, die Funktionaliserung über den Regler am Kettenende. Um eine Funktionalisierung zu erreichen, muss daher mindestens ein Hydroxyl-Gruppen tragender Radikalstarter oder ein Hydroxyl-Gruppen tragender Regler bei der Polymerisation der Präpolymeren eingesetzt werden. Sollen B-A-B- oder A-B-A-Blockcopolymere hergestellt werden, müssen Radikalstarter und Regler Hydroxyl-Gruppen tragen.

Allgemeine Verfahren zur Herstellung der Vinyllactam-Präpolymeren und der Polyvinylacetat-Präpolymeren sind an sich bekannt.

Die Herstellung erfolgt durch radikalisch initiierte Poylmerisation in geigneten Lösungsmitteln.

Als N-Vinyllactame eignen sich N-Vinylpyrrolidon, N-Vinylcaprolactam oder N-Vinylpiperidon oder Mischungen davon. Bevorzugt wird N-Vinylpyrrolidon eingesetzt.

Geeignete nichtwässrige Lösungsmittel sind beispielsweise Alkohole, wie Methanol, Ethanol, n-Propanol, und Isopropanol sowie Glykole, wie Ethylenglykol und Glycerin.

Weiterhin eignen sich als Lösungsmittel Essigsäureester wie beispielsweise Ethylacetat oder Butylacetat.

Bevorzugt werden solche Lösungsmitel eingesetzt, die nicht als Regler wirken. Dem Fachmann sind diese bekannt.

Für die Lösungsmittel zur Herstellung des Polyvinylacetats gilt das oben gesagte.

Die Polymerisation wird vorzugsweise bei Temperaturen von 60 bis 100°C durchgeführt.

Zur Initiierung der Polymerisation werden radikalische Initiatoren als Radikalstarter eingesetzt. Die verwendeten Mengen an Initiator bzw. Initiatorgemischen bezogen auf eingesetztes Monomer liegen zwischen 0,01 und 10 Gew.-%, vorzugsweise zwischen 0,3 und 5 Gew.-%.

Je nach Art des verwendeten Lösungsmittels eignen sich sowohl organische als auch anorganische Peroxide wie Natriumpersulfat oder Azostarter wie Azo-bis-isobutyronitril, Azo-bis-(2-amidopropan)dihydrochlorid oder 2,2'-Azo-bis-(2-methyl-butyronitril).

Peroxidische Initiatoren sind beispielsweise Dibenzoylperoxid, Diacetylperoxid, Succinylperoxid, tert.-Butylperpivalat, tert.-Butyl-2-ethylhexanoat, tert.-Butylpermaleinat, Bis-(tert.-Butylperoxi)-cyclohexan, tert.-Butylperoxi-isopropylcarbonat, tert.-Butylperacetat, 2,2-Bis-(tert.-butylperoxi)-butan, Dicumylperoxid, Di-tert.-amylperoxid, Di-tert.-butylperoxid, p-Menthanhydroperoxid, Pinanhydroperoxid, Cumolhydroperoxid, tert.-Butylhydroperoxid, Wasserstoffperoxid sowie Mischungen der genannten Initiatoren. Die genannten Initiatoren können auch in Kombination mit Redoxkomponenten wie Ascorbinsäure verwendet werden.

Soll die OH-Funktionalisierung über den Radikalstarter erfolgen, eignen sich insbesondere OH-funktionalisierte Starter, wie beispielsweise 2,2'-Azobis[2-methyl-N-(2-hydroxyethyl)propionamid], 2,2'-Azobis{2-methyl-N-[2-(1-hydroxybuthyl)]propionamide} oder 2,2'-Azobis{2-[1-(2-hydroxyethyl)-2-imidazolin-2-yl]propane}dihydrochloride.

Die radikalische Polymerisation kann gegebenenfalls in Gegenwart von Emulgatoren, gegebenenfalls weiteren Schutzkolloiden, gegebenenfalls Puffersystemen und gegebenenfalls nachfolgender pH-Einstellung mittels Basen oder Säuren statt.

Als Molekulargewichtsregler eignen sich Schwefelwasserstoffverbindungen wie Alkylmercaptane, z.B. n-Dodecylmercaptan, tert.-Dodecylmercaptan, Thioglykolsäure und deren Ester, Mercaptoalkanole wie Mercaptoethanol. Weitere geeignete Regler sind z.B. in der DE 197 12 247 A1, Seite 4, genannt. Die erforderliche Menge der Molekulargewichtsregler liegt im Bereich von 0 bis 5 Gew.-% bezogen auf die zu polymerisierenden Monomerenmenge, insbesondere 0,05 bis 2 Gew.-%, besonders bevorzugt 0,1 bis 1,5 Gew.-%. Bevorzugt wird Mercaptoethanol eingesetzt.

Das Monomere bzw. eine Monomerenmischung oder die Monomer(en)emulsion werden zusammen mit dem Initiator, der üblicherweise in Lösung vorliegt, in einem Rührreaktor bei der Polymerisationstemperatur vorgelegt (Batch-Prozess), oder gegebenenfalls kontinuierlich oder in mehreren aufeinanderfolgenden Stufen in den Polymerisationsreaktor zudosiert (Zulaufverfahren). Beim Zulaufverfahren ist es üblich, dass der Reaktor vor Beginn der eigentlichen Polymerisation neben Wasser (um eine Rührung des Reaktors zu ermöglichen) bereits mit Teilmengen, selten der gesamten für die Polymerisation vorgesehenen Menge, der Einsatzstoffe wie Emulgatoren, Schutzkolloiden, Monomeren, Regler usw. oder Teilmengen der Zuläufe (i.a. Monomer- oder Emulsionszulauf sowie Initiatorzulauf) befüllt wird.

Die Polyvinylacetate werden äquimolar, bezogen auf die Hydroxyl-Gruppen im Polyvinylacetat und im Vinyllactam-Präpolymer, umgesetzt. Die Menge der vorhandenen OH-Gruppen kann, soweit erforderlich, auf dem Fachmann an sich bekannte Weise ermittelt werden. Zur Ermittlung der Hydroxyl-Zahl siehe beispielsweise Römpp Chemie Lexikon, 9. Auflage, 1990.

Die Kupplung von Vinyllactampolymeren und Polyvinylacetaten erfolgt durch Umsetzung mit Diisocyanaten, wobei es durch Reaktion mit den Hydroxyl-Gruppen des Polymers zu einer Ankupplung über Urethan-Gruppen kommt. Dabei kann entweder das Vinyllactam-polymer oder das Polyvinylacetat zunächst mit dem Diisocyanat umgesetzt werden.

Gemäß einer bevorzugten Ausführungsform der Erfindung erfolgt die Ankupplung über mit Isocyanatgruppen als Endgruppen funktionalisierten Polyvinylacetaten. Dazu wird zunächst das Polyvinylacetat mit dem Diisocyanat umgesetzt und anschliessend das so funktionalisierte Polyvinylacetat mit dem Vinyllactampolymer umgesetzt.

Unabhängig davon, welche Ausführungsform gewählt wird, kann die Umsetzung wie folgt erfolgen:
Als Diisocyanate kommen Verbindungen der allgemeinen Formel OCN-R-NCO, wobei R aliphatische, alicyclische oder aromatische Reste, die auch mit Alkylresten substituiert sein können, bedeuten kann, in Betracht.

Als Diisocyanate eignen sich bevorzugt Verbindungen, deren Isocyanat-Gruppen aufgrund der Molekülstruktur eine unterschiedliche Reaktivität gegenüber Nucleophilen aufweisen, beispielsweise Isophorondiisocyanat oder Toluylendiisocyanat.

Grundsätzlich eignen sich auch symmetrische Diisocyanate wie beispielsweise Hexamethylendisocyanat oder 4,4'-Methylendi-(phenylisocyanat).

Bevorzugt wird Isophorondiisocyanat eingesetzt.

Die Umsetzung mit dem Diisocyanat erfolgt vorzugsweise in einem organischen Lösungsmittel wie Ketonen, beispielsweise Aceton, weiterhin Dimethylsulfoxid, Dimethylformamid, oder allgemein aprotisch-polaren organischen Lösungsmitteln oder Gemischen solcher Lösungsmittel. Die Umsetzung erfolgt üblicherweise bei erhöhten Temperaturen, wobei sich dieTemperatur auch nach der Siedetemperatur des gewählten Lösungsmittels richtet. Die Umsetzung des Diisocyanats mit der ersten Komponente kann bei 20 bis 50 °C, gewünschtenfalls aber auch bis 100 °C erfolgen. Die Umsetzung der zweiten Isocyanatgruppe kann bei Temperaturen von 50 bis 100 °C erfolgen.

Die Umsetzung erfolgt vorzugsweise äquimolar, was bedeutet, dass das Mengenverhältnis so gewählt wird, dass pro Mol umzusetzender Hydroxyl- Gruppe 1 Mol Diisocyanat eingesetzt wird. Ist das Vinyllactampolymer über einen Regler OH-funktionalisiert, wird das Diisocyanat äquimolar zum Regler umgesetzt. Ist das Vinyllactampolymer über einen Radikalstarter OH-funktionalisiert , so werden pro Mol Radikalstarter 2 Mol Diisocyanat eingesetzt.

Im Falle symmetrischer Diisocyanate kann es sich auch empfehlen, einen Überschuss an Diisocyanat einzusetzen und den Überschuss anschliessend destillativ zu entfernen.

Vorzugsweise wird die Umsetzung in Gegenwart eines Katalysators durchgeführt. Als Katalysatoren eignen sich beispielsweise metallorganische Verbindungen wie Organotitan-Verbindungen oder Zinkverbindungen wie Dibutylzinndilaurat oder Zinnoctoat, weiterhin Basen wie 1,4-Diaza(2,2,2)bicyclooctan oder Tetramethylbutandiamin. Der Katalysator kann in Mengen von 0,05 bis 0,2 Mol, vorzugsweise 0,1 bis 0,14 Mol, pro Mol Diisocyanat eingesetzt werden.

Die Umsetzung wird üblicherweise bei erhöhten Temperaturen im Bereich von 50 bis 100 °C durchgeführt. Welche Temperatur im Einzelnen gewählt wird, hängt von der Art des verwendeten organischen Lösungsmittels ab. Das Lösungsmittel kann anschliessend durch Destillation entfernt werden.

Üblicherweise wird man die Umsetzung so durchführen, dass zunächst die Komponente, die isocyanatgruppenfunktionalisiert werden soll, mit dem Diisocyanat in Gegenwart des Katalysators und eines Lösungsmittels solange umgesetzt wird, bis der Isocyanat-Wert im Reaktionsgemisch auf die Hälfte gesunken ist. Dies lässt sich auf bekannte Weise beispielsweise titrimetrisch ermitteln. Danach erfolgt dann die Zugabe der anderen Komponente, wobei die Mengen an Isocyantgruppen und OH- oder Aminogruppen wiederum äquimolar gewählt werden. Die Reaktion wird fortgeführt, bis der Isocyanatwert auf Null gesunken ist.

Bevorzugt werden die Blockcopolymere durch ein an sich bekanntes Verfahren der kontrollierten radikalischen Polymerisation, das auch als RAFT-Polymerisation (Reversible Addition -Fragmentation Chain Transfer) bezeichnet wird, hergestellt. Der Mechanismus dieses Verfahrens ist ausführlich in der WO 98/01478 und der EP-A 991 683 beschrieben, auf deren Offenbarung hiermit ausdrücklich Bezug genommen wird.

Die RAFT-Polymerisation erfolgt in Gegenwart spezifischer Kettenübertragungsmittel, auch als RAFT-Reagentien bezeichnet, aus der Gruppe der Thiocarbonylthioverbindugen, insbesondere der Dithiobenzoate, der Trithiocarbonate, der Dithiocarbamate und der Dithiokohlensäureester, die auch als Xanthate bezeichnet werden. Solche Reagentien sind dem Fachmann aus dem Stand der Technik bekannt. So eignen sich beispielsweise die in der WO 98/01478 oder der EP-A 991 683 beschriebenen Verbindungen.

Bevorzugte RAFT-Reagentien sind Diphenyldithiocarbamat des Dieethylmalonats und 2-(ethoxycarbonothioyl)thiopropionsäure.

Zur Herstellung des Blockcopolymers wird zunächst der PVP-Block durch radikalisch initiierte Lösungspolymerisation hergestellt, indem N-Vinyllactam, RAFT-Reagenz und Radikalstarter in einem Lösungsmittel gemischt und bei erhöhter Temperatur umgesetzt werden.

Als Radikalstarter eignen sich Azostarter wie 2,2'-Azobis(2,4-dimethyl-valeronitril), Dimethyl, Azobis-isobutyronitril, Dimethyl-2,2'-azobis-isobutyrat, 1,1'-Azobis(1-cyclohexancarbonitril), 2,2'-Azobis(2-methylbutyronitril) oder 4,4'-Azobis[2-methyl-N-2-hydroxyethyl)propionamid], vorzugsweise Azobisisobutyronitril (AIBN). Der Radikalstarter kann in Mengen von 5 bis 50 mol-%, bevorzugt 5 bis 15 mol-%, bezogen auf RAFT-Reagenz, eingesetzt werden.

Die Vinyllactammonomere, RAFT-Reagentien und Radikalstarter können in Molverhältnissen von 350 :2 : 1 (Monomer:RAFT:Starter) bis 3500 : 10 : 1 eingesetzt werden, bevorzugt 1000:10:1 bis 2000:10:1.

Als Lösungsmittel eignen sich organische Lösungsmittel, die nicht als Regler wirken, beispielsweise Methanol, n-Propanol, tert-Butanol, Dimethylformamid, Ethylacetat, Butylacetat oder Dioxan, bevorzugt Dioxan oder tert-Butanol.

Die Umsetzung kann bei Temperaturen von 50 bis 120 °C, vorzugsweise 60 bis 80 °C erfolgen.

Nach Beendigung der Polymerisation empfiehlt es sich, das mit RAFT-Reagenz funktionalisierte Polyvinyllactam so zu behandlen, dass gegebenenfalls nicht umgesetztes Vinyllactam entfernt wird. Dies kann beispielsweise erfolgen, indem das Polymer aus der Reaktionsmischung ausgefällt und durch Filtration abgetrennt wird. Die Fällung kann beispielsweise durch Zugabe eines Nichtlösemittels erfolgen. Als Nichtlösemittel eignet sich vor allem Diethylether. Weiterhin kann das Polymer auch durch saure Hydrolyse des monomeren Vinyllactams mit anschliessender Entfernung des entstehenden Lactams durch Destillation aufgereinigt werden.

Der mit dem RAFT-Reagenz funktionalisierte PVP-Block wird anschliessend mit Vinylacetat in Gegenwart eines Radikalstarters umgesetzt.

Als Radikalstarter eignen sich die für die Herstellung des Polyvinyllactam-Blocks beschriebenen Verbindungen. Die Umsetzung kann ansonsten unter den für die Bildung des Polyvinyllactam-Blocks beschriebenen Bedingungen erfolgen.

Nach Beendigung der Polymerisation kann das resultierende Blockpolymer auf an sich übliche Weise aufgearbeitet werden, beispielsweise durch destillative Abtrennung des Lösungsmittels.

Die Blockcopolymere sind bevorzugt wasserlöslich, können aber auch wasserdispergierbar sein.

Die Molekulargewichte Mn können 5000 bis 50.000, vorzugsweise 10.000 bis 30.000 betragen.

### Anwendungen:

Der Begriff "in Wasser schwerlöslich" umfasst erfindungsgemäß auch praktisch unlösliche Substanzen und bedeutet, dass für eine Lösung der Substanz in Wasser bei 20 °C mindestens 30 bis 100 g Wasser pro g Substanz benötigt wird. Dies bedeutet beispielsweise, dass mindestens 30 g, in vielen Fällen aber auch mindestens 100 g Wasser pro g Substanz benötigt wird. Bei praktisch unlöslichen Substanzen werden mindestens 10.000 g Wasser pro g Substanz benötigt. Es kommen erfindungsgemäß agrochemische Wirkstoffe zur Behandlung gegen Schadorganismen wie beispielsweise Insekticide, Herbicide oder Fungicide sowie Pflanzenwuchsstoffe oder Mittel zur Saatgutbehandlung in Betracht.

Des weiteren werden durch die vorliegende Erfindung insbesondere amphiphile Verbindungen für die Anwendung als Kristallisationsinhibitor für agrochemische Zubereitungen zur Verfügung gestellt.

Sie besitzen die Eigenschaft, die Kristallisation von schwer löslichen Wirkstoffen für den Einsatz in Pflanzenschutzmitteln (auch Pestizide oder agrochemische Wirkstoffe genannt) zu inhibieren.

### Solubilisatoren für agrochemische Wirkstoffe (Pestizide):

Gegenstand der vorliegenden Erfindung sind daher auch agrochemische Zubereitungen, die mindestens eines der erfindungsgemäßen Copolymere als Kristallisationsinhibitoren enthalten und mindestens ein schwerlösliches Pestizid.

Der Begriff "schwerlösliches Pestizid" bezeichnet hier ein in Wasser schwerlösliches Pestizid. Hierbei umfasst, wie bereits oben erwähnt der Begriff "in Wasser schwerlöslich" erfindungsgemäß auch praktisch unlösliche Substanzen und bedeutet, dass für eine Lösung des Pestizides in Wasser bei 20 °C mindestens 30 bis 100 g Wasser pro g Pestizid benötigt wird, vorzugsweise mindestens 100g Wasser pro 1g Pestizid. Bei praktisch unlöslichen Pestiziden werden mindestens 10.000 g Wasser pro g Substanz benötigt.

Pestizide bzw. agrochemische Wirkstoffe sind dem Fachmann aus der Literatur bekannt. Der Begriff "Pestizid" bedeutet hier mindestens ein Wirkstoff ausgewählt aus der Gruppe der Insektizide, Fungizide, Herbizide und/oder Safener (s. Pesticide Manual, 13th Ed. (2003)) verwendet wird.

Beispiele für schwerlösliche Pestizide sind Insektizide, Fungizide, Herbizide und/oder Safener sind im folgenden aufgeführt:
Die folgende Liste von schwerlöslichen Insektiziden zeigt mögliche Wirkstoffe auf, soll aber nicht auf diese beschränkt sein:
   A.1. Organo(thio)phosphate: azinphos-methyl, chlorpyrifos, chlorpyrifos-methyl, chlorfenvinphos, diazinon, disulfoton, ethion, fenitrothion, fenthion, isoxathion, malathion, methidathion, methyl-parathion, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   A.2. Carbamate: alanycarb, bendiocarb, benfuracarb, carbaryl, carbofuran, carbosulfan, fenoxycarb, furathiocarb, methiocarb, methomyl, oxamyl, pirimicarb, thiodicarb, triazamate;
   A.3. Pyrethroide: allethrin, bifenthrin, cyfluthrin, cyhalothrin, cyphenothrin, cyper-methrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin;
   A.4. Wachstumsregulatoren: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, a tetronic acid derivative of formula Γ¹, 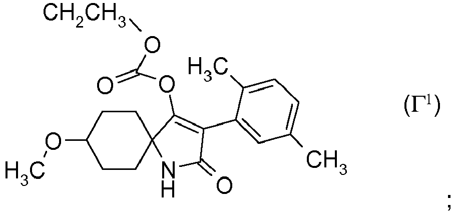
   A.5. Nicotin Rezeptor Agonisten/ Antagonisten: clothianidin, dinotefuran, thiacloprid;
   A.6. GABA Antagonisten: acetoprole, endosulfan, ethiprole, fipronil, vaniliprole;
   A.7. Macrolid-Insektizide: abamectin, emamectin, milbemectin, lepimectin, spinosad;
   A.8. METI I Acarizide: fenazaquin, pyridaben, tebufenpyrad, tolfenpyrad;
   A.9. METI II and III Verbindung: acequinocyl, fluacyprim, hydramethylnon;
   A.10. Entkoppler-Verbindungen: chlorfenapyr;
   A.11. Hemmer der oxidativen Phosphorylierung: cyhexatin, diafenthiuron, fenbutatin oxide, propargite;
   A.12. Häutungsstörende Verbindungen: cryomazine;
   A.13. Hemmer der Mixed-Function-Oxidase: piperonyl butoxide;
   A.14. Natriumkanalblocker: indoxacarb, metaflumizone;
   A.15. Verschiedene: benclothiaz, bifenazate, flonicamid, pyridalyl, pymetrozine, sulfur, thiocyclam und aminoisothiazole Verbindungen der Formel Γ², 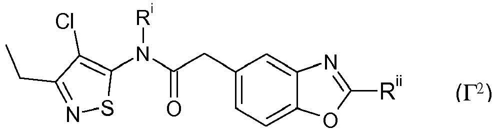 wobei Rⁱ für -CH₂OCH₂CH₃ or H und Rⁱⁱ für CF₂CF₂CF₃ or CH₂CH(CH₃)₃ steht, anthranilamide Verbindungen der Formel Γ³ 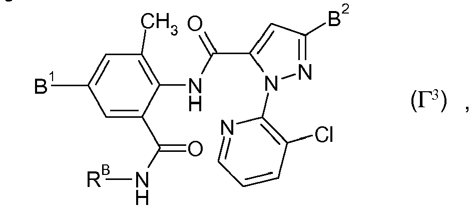 wobei B1 für Wasserstoff oder Chlor, B2 für Brom oder CF3, und RB für CH3 oder CH(CH3)2 steht, und malononitrile Verbindungen wie in JP 2002 284608, WO 02/89579, WO 02/90320, WO 02/90321, WO 04/06677, WO 04/20399, oder JP 2004 99597 beschrieben, N-R'-2,2-dihalo-1-R"cyclo-propanecarboxamide-2-(2,6-dichloro- α,α,α,α -tri-fluoro-p-tolyl)hydrazone oder N-R'-2,2-di(R"')propionamide-2-(2,6-dichloro- α,α,α,α -trifluoro-p-tolyl)-hydrazone, worin R' für methyl oder ethyl stehen, halo für Chlor oder Brom steht, R" für Wasserstoff oder methyl und R'" für methyl oder ethyl stehen;

Die folgende Liste von schwerlöslichen Fungiziden zeigt mögliche Wirkstoffe auf, soll aber nicht auf diese beschränkt sein:
1. Strobilurine
   Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-((2,5-Dimethylphenyl-oxymethylen)phenyl)-3-methoxy-acrylsäuremethylester;
2. Carbonsäureamide
   - Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure (2-cyano-phenyl) amid; 3-Difluormethyl-1-methyl-pyrazol-4-carbonsäure-(3',4'-dichlor-5-fluor-biphenyl-2-yl)-amid, 3-Difluormethyl-1-methyl-1 H-pyrazol-4-carbonsäure-(2-bicyclopropyl-2-yl-phenyl)amid);
      in einer weiteren Ausführungsform sind Beispiele für Carbonsäureanilide Benalaxyl-M, Bixafen, Isotianil, Kiralaxyl, Tecloftalam, 2-Amino-4-methyl-thiazol-5-carbonsäureanilid, 2-Chlor-N-(1,1,3-trimethyl-indan-4-yl)-nicotinamid, N-(3',4'-Dichlor-5-fluor-biphenyl-2-yl)-3-difluormethyl-1-methyl-1 H-pyrazol-4-carbonsäureamid, 5-Fluor-1,3-dimethyl-1 H-pyrazol-4-carbonsäure [2-(1,3-dimethyl-butyl)-phenyl]-amid, N-(4'-Chlor-3',5-difluor-bi-phenyl-2-yl)-3-difluormethyl-1-methyl-1 H-pyrazol-4-carbonsäureamid, N-(4'-Chlor-3',5-difluor-biphenyl-2-yl)-3-trifluormethyl-1-methyl-1 H-pyrazol-4-carbonsäureamid, N-(3',4'-Dichlor-5-fluor-biphenyl-2-yl)-3-trifluormethyl-1-methyl-1 H-pyrazol-4-carbonsäureamid, N-(3',5-Difluor-4'-methyl-biphenyl-2-yl)-3-difluormethyl-1-methyl-1H-pyrazol-4-carbonsäureamid, N-(3',5-Difluor-4'-methyl-biphenyl-2-yl)-3-trifluormethyl-1-methyl-1H-pyrazol-4-carbonsäureamid, N-(2-Bicyclopropyl-2-yl-phenyl)-3-difluormethyl-1-methyl-1 H-pyrazol-4-carbonsäureamid, N-(cis-2-Bicyclopropyl-2-yl-phenyl)-3-difluormethyl-1-methyl-1 H-pyrazol-4-carbonsäureamid, N-(trans-2-Bicyclopropyl-2-yl-phenyl)-3-difluormethyl-1-methyl-1 H-pyrazol-4-carbonsäureamid;
   - Carbonsäuremorpholide: Dimethomorph, Flumorph;
   - Benzoesäureamide: Flumetover, Fluopicolide (Picobenzamid), Zoxamide; in einer weiteren Ausführungsform ist ein Beispiel für Benzoesäureamide N-(3-Ethyl-3,5-5trimethyl-cyclohexyl)-3-formylamino-2-hydroxy-benzamid;
   - Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid, 3-Difluormethyl-1-methyl-pyrazol-4-carbonsäure-(3',4'-dichlor-5-fluor-biphenyl-2-yl)-amid und 3-Difluormethyl-1-methyl-1 H-pyrazol-4-carbonsäure-(2-bicyclopropyl-2-yl-phenyl)amid); in einer weiteren Ausführungsform sind Beispiele für sonstige Carbonsäureamide Oxytetracyclin, Silthiofam, N-(6-methoxy-pyridin-3-yl)cyclopropancarbonsäureamid.
3. Azole
   - Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazole, Myclobutanil, Penconazole, Propiconazole, Prothioconazole, Simeconazole, Tebuconazole, Tetraconazole, Triadimenol, Triadimefon, Triticonazole;
   - Imidazole: Cyazofamid, Imazalil, Pefurazoate, Prochloraz, Triflumizole;
   - Benzimidazole: Benomyl, Carbendazim, Fuberidazole, Thiabendazole;
   - Sonstige: Ethaboxam, Etridiazole, Hymexazole;
4. Stickstoffhaltige Heterocyclylverbindungen:
   - Pyridine: Fluazinam, Pyrifenox, 3-[5-(4-Chlor-phenyl)-2,3-dimethyl-isoxazolidin-3-yl]-pyridin;
   - Pyrimidine: Bupirimate, Cyprodinil, Ferimzone, Fenarimol, Mepanipyrim, Nuarimol, Pyrimethanil;
   - Piperazine: Triforine;
   - Pyrrole: Fludioxonil, Fenpiclonil;
   - Morpholine: Aldimorph, Dodemorph, Fenpropimorph, Tridemorph;
   - Dicarboximide: Iprodione, Procymidone, Vinclozolin;
   - sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Quinoxyfen, Tricyclazole, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;
5. Carbamate und Dithiocarbamate
   - Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäure-methylester, N-(1-(1-(4-cyanophenyl)ethansulfonyl)-but-2-yl) carbaminsäure-(4-fluor-phenyl)ester;
6. Sonstige Fungizide
   - Organometallverbindungen: Fentin Salze;
   - Schwefelhaltige Heterocyclylverbindungen: Isoprothiolane, Dithianon;
   - Organophosphorverbindungen: Edifenphos, Fosetyl, Fosetyl-aluminium, Iprobenfos, Pyrazophos, Tolclofos-methyl, Phosphorige Säure und ihre Salze;
   - Organochlorverbindungen: Thiophanate Methyl, Chlorothalonil, Dichlofluanid, Tolylfluanid, Flusulfamide, Phthalide, Hexachlorbenzene, Pencycuron, Quintozene;
   - Nitrophenylderivate: Binapacryl, Dinocap, Dinobuton;
   - Sonstige: Spiroxamine, Cyflufenamid, Cymoxanil, Metrafenone.

Die folgende Liste von schwerlöslichen Herbiziden zeigt mögliche Wirkstoffe auf, soll aber nicht auf diese beschränkt sein:
Verbindungen, die die Biosynthese von Lipiden inhibieren, z.B. Chlorazifop, Clodinafop, Clofop, Cyhalofop, Ciclofop, Fenoxaprop, Fenoxaprop-p, Fenthiaprop, Fluazifop, Fluazifop-P, Haloxyfop, Haloxyfop-P, Isoxapyrifop, Metamifop, Propaquizafop, Quizalofop, Quizalofop-P, Trifop, bzw. deren Esters, Butroxydim, Cycloxydim, Profoxydim, Sethoxydim, Tepraloxydim, Tralkoxydim, Butylate, Cycloat, Diallat, Dimepiperat, EPTC, Esprocarb, Ethiolate, Isopolinate, Methiobencarb, Molinate, Orbencarb, Pebulate, Prosulfocarb, Sulfallat, Thiobencarb, Thiocarbazil, Triallat, Vernolat, Benfuresat, Ethofumesat und Bensulid;
ALS-Inhibitoren wie Amidosulfuron, Azimsulfuron, Bensulfuron, Chlorimuron, Chlorsulfuron, Cinosulfuron, Cyclosulfamuron, Ethametsulfuron, Ethoxysulfuron, Flazasulfuron, Flupyrsulfuron, Foramsulfuron, Halosulfuron, Imazosulfuron, lodosulfuron, Mesosulfuron, Metsulfuron, Nicosulfuron, Oxasulfuron, Primisulfuron, Prosulfuron, Pyrazosulfuron, Rimsulfuron, Sulfometuron, Sulfosulfuron, Thifensulfuron, Triasulfuron, Tribenuron, Trifloxysulfuron, Triflusulfuron, Tritosulfuron, Imazamethabenz, Imazamox, Imazapic, Imazapyr, Imazaquin, Imazethapyr, Cloransulam, Diclosulam, Florasulam, Flumetsulam, Metosulam, Penoxsulam, Bispyribac, Pyriminobac, Propoxycarbazone, Flucarbazone, Pyribenzoxim, Pyriftalid und Pyrithiobac; sofern der pH Wert < 8 ist
Verbindungen, die die Photosynthese inhibieren wie Atraton, Atrazine, Ametryne, A-ziprotryne, Cyanazine, Cyanatryn, Chlorazine, Cyprazine, Desmetryne, Dimethametryne, Dipropetryn, Eglinazine, Ipazine, Mesoprazine, Methometon, Methoprotryne, Procyazine, Proglinazine, Prometon, Prometryne, Propazine, Sebuthylazine, Secbumeton, Simazine, Simeton, Simetryne, Terbumeton, Terbuthylazine und Terbutryne;
Protoporphyrinogen-IX Oxidase-Inhibitoren wie Acifluorfen, Bifenox, Cchlomethoxyfen, Chlornitrofen, Ethoxyfen, Fluorodifen, Fluoroglycofen, Fluoronitrofen, Fomesafen, Furyloxyfen, Halosafen, Lactofen, Nitrofen, Nitrofluorfen, Oxyfluorfen, Fluazolate, Pyraflufen, Cinidon-ethyl, Flumiclorac, Flumioxazin, Flumipropyn, Fluthiacet, Thidiazimin, O-xadiazon, Oxadiargyl, Azafenidin, Carfentrazone, Sulfentrazone, Pentoxazone, Benzfendizone, Butafenacil, Pyraclonil, Profluazol, Flufenpyr, Flupropacil, Nipyraclofen und Etnipromid;
Herbizide wie Metflurazon, Norflurazon, Flufenican, Diflufenican, Picolinafen, Beflubutamid, Fluridone, Flurochloridone, Flurtamone, Mesotrione, Sulcotrione, Isoxachlortole, Isoxaflutole, Benzofenap, Pyrazolynate, Pyrazoxyfen, Benzobicyclon, amitrole, clomazone, Aclonifen, 4-(3-trifluormethylphenoxy)- 2-(4-trifluoromethylphenyl)pyrimidin, und 3-heterocyclyl-substituierte Benzoylderivate der Formel (vgl. WO-A-96/26202, WO-A-97/41116, WO-A-97/41117 und WO-A-97/41118) 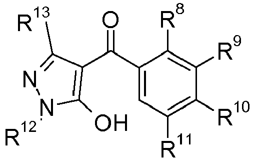 worin die Substituenten R⁸ to R¹³ folgende Bedeutung haben:
   R⁸, R¹⁰ Wasserstoff, Halogen, C₁-C₆-Alkyl, C₁-C₆-Haloalkyl, C₁-C₆-Alkoxy, C₁-C₆-Haloalkoxy, C₁-C₆-Alkylthio, C₁-C₆-Alkylsulfinyl oder C₁-C₆-Alkylsulfonyl;
   R⁹ bedeutet ein heterocyclisches Radikal aus der Gruppe bestehend aus Thiazol-2-yl, thiazol-4-yl, Thiazol-5-yl, Isoxazol-3-yl, Isoxazol-4-yl, Isoxazol-5-yl, 4,5-dihydroisoxazol-3-yl, 4,5-dihydroisoxazol-4-yl und 4,5-dihydroisoxazol-5-yl, worin die genannten Radikale einen oder mehrere Substituenten tragen können z.B. mono-, di-, tri- or tetrasubstituiert sein können durch Halogen, C₁-C₄-Alkyl, C₁-C₄-Alkoxy, C₁-C₄-Haloalkyl, C₁-C₄-Haloalkoxy oder C₁-C₄-Alkylthio;
   R¹¹ = Wasserstoff, Halogen oder C₁-C₆-Alkyl;
   R¹² = C₁-C₆-Alkyl;
   R¹³ = Wasserstoff oder C₁-C₆-Alkyl.

Sofern der pH Wert < 8 ist.

Mitose-Inhibitoren wie Benfluralin, Butralin, Dinitramine, Ethalfluralin, Fluchloralin, i-Sopropalin, Methalpropalin, Nitralin, Oryzalin, Pendimethalin, Prodiamine, Profluralin, Trifluralin, Amiprofos-methyl, Butamifos, Dithiopyr, Thiazopyr, Propyzamide, Chlorthal, Carbetamide, Chlorpropham and Propham;
VLCFA-Inhibitoren wie Acetochlor, Alachlor, Butachlor, Butenachlor, Delachlor, Diethatyl, Dimethachlor, Dimethenamid, Dimethenamid-P, Metazachlor, Metolachlor, S-Metolachlor, Pretilachlor, Propisochlor, Prynachlor, Terbuchlor, Thenylchlor, Xylachlor, CDEA, Epronaz, Diphenamid, Napropamide, Naproanilide, Pethoxamid, Flufenacet, Mefenacet, Fentrazamide, Anilofos, Piperophos, Cafenstrole, Indanofan und Tridiphan;
Inhibitoren für die Biosynthese von Cellulose wie Dichlobenil, Chlorthiamid, Isoxaben und Flupoxam;
Herbizide wie Dinofenat, Dinoprop, Dinosam, Dinoseb, Dinoterb, DNOC, Etinofen und Medinoterb;
außerdem: Benzoylprop, Flamprop, Flamprop-M, Bromobutide, Chlorflurenol, Cinmethylin, Methyldymron, Etobenzanid, Pyributicarb, Oxaziclomefone, Triaziflam und Methyl bromide.

Die folgende Liste zeigt mögliche schwerlösliche Safener auf, soll aber nicht auf diese beschränkt sein:
benoxacor, cloquintocet, cyometrinil, dicyclonon, dietholate, fenchlorazole, fenclorim, flurazole, fluxofenim, furilazole, isoxadifen, mefenpyr, mephenate, naphthalic anhydride, 2,2,5-trimethyl-3-(dichloroacetyl)-1,3-oxazolidine (R-29148), 4-(dichloroacetyl)-1-oxa-4-azaspiro[4.5]decane (AD-67; MON 4660) und oxabetrinil.

Bevorzugte Fungizide sind Triazole wie Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazole, Myclobutanil, Penconazole, Propiconazole, Prothioconazole, Simeconazole, Tebuconazole, Tetraconazole, Triadimenol, Triadimefon, Triticonazole, Strobilurine wie sowie Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-((2,5-Dimethylphenyl-oxymethylen)phenyl)-3-methoxy-acrylsäuremethylester sowie 5-Chlor-7-(4-methyl-piperidin-1-yl)-6-(2,4,6-trifluor-phenyl)-[1,2,4]triazolo[1,5-a]pyrimidin und boscalid.

Ganz besonders bevorzugte Fungizide sind Epoxiconazole, Metconazole, Pyraclostrobin, Kresoxim-methyl und 5-Chlor-7-(4-methyl-piperidin-1-yl)-6-(2,4,6-trifluor-phenyl)-[1,2,4]triazolo[1,5-a]pyrimidin und boscalid.

Bevorzugte Insektizide sind metaflumizon, fipronil und alpha-cypermethrin.

In einer weiteren bevorzugten Ausführungsform sind Mischungen umfassend mindestens zwei verschiedene Triazole bevorzugt, insbesondere Mischungen umfassend Metconazol und Epoxiconazol, Metconazol und Prothioconazol, oder Epoxiconazol und Prothioconazol.

In einer weiteren bevorzugten Ausführungsform sind Mischungen umfassend mindestens ein Triazol und mindestens ein Strobilurin bevorzugt. Speziell sind Mischungen umfassend Pyraclostrobin und Epoxiconazol sowie Mischungen umfassend Pyraclostrobin und Metconazol bevorzugt.
In einer weiteren bevorzugten Ausführungsform sind Mischungen umfassend mindestens ein Triazol und mindestens ein Carbonsäureamid bevorzugt. Insbesondere sind Mischungen bevorzugt, die mindestens ein Triazol, mindestens ein Carbonsäureamid und mindestens ein Strobilurin enthalten.

In den agrochemischen Zubereitungen beträgt das Massenverhältnis von Polymer:Wirkstoff verhältnis 1:10 (w/w) bis 100:1 (w/w), bevorzugt 1:2(w/w) bis 50:1 (w/w), besonders bevorzugt 1:1 (w/w) bis 10:1 (w/w), besonders besonders bevorzugt 2:1 (w/w) bis 10:1 (w/w).

Die agrochemischen Zubereitungen können weiterhin auch für die Formulierung von Pestiziden übliche Hilfsmittel enthalten, wobei sich die Wahl der Hilfsmittel nach der konkreten Anwendungsform bzw. dem Wirkstoff richtet.

In der Regel liegt die Menge an eingesetzten Hilfsmitteln zwischen 0 bis 60 %Gew , vorzugsweise 0,1 bis 30 Gew %.

Beispiele für die Formulierung von Pestiziden geeignete Hilfsmittel sind Lösungsmittel, feste Trägerstoffe, oberflächenaktive Stoffe (wie weitere Solubilisatoren, Schutzkolloide, Netzmittel und Haftmittel), organische und anorganische Verdicker, Bakterizide, Frostschutzmittel, Entschäumer.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide sowie organische und anorganische Schichtmineralien wie Xanthan Gum (Kelzan® der Fa. Kelco), Rhodopol® 23 (Rhone Poulenc) oder Veegum® (Firma R.T. Vanderbilt) oder Attaclay® (Firma Engelhardt).

Beispiele für Antischaummittel sind Silikonemulsionen (wie z.Bsp. Silikon® SRE, Firma Wacker oder Rhodorsil® der Firma Rhodia), langkettige Alkohole, Fettsäuren, Salze von Fettsäuren, fluororganische Verbindungen und deren Gemische.

Beispiele für Bakterizide sind Bakterizide basierend auf Diclorophen und Benzylalkoholhemiformal (Proxel® der Fa. ICI oder Acticide® RS der Fa. Thor Chemie und Kathon® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Als Lösungsmittel kommen organische Lösungsmittel wie Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon sowie Gemische aus den vorstehend genannten Lösungsmitteln und Wasser, sowie Gemische aus Wasser und organischen Lösungsmitteln in Betracht.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Als oberflächenaktive Stoffe (Adjuvantien, Netz-, Haft-, Dispergier- oder Emulgiermittel) kommen die Alkali-, Erdalkali-, Ammoniumsalze von aromatischen Sulfonsäuren, z.B. Lignin-(Borresperse typen Borregaard), Phenol-, Naphthalin-(Morwet typen, Akzo Nobel) und Dibutylnaphthalinsulfonsäure (Nekal typen BASF), sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol" Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugensowie Proteine, denaturierte Proteine, Polysaccharide (z.B. Methylcellulose), hydrophob modifizierte Stärken, Polyvinylalkohol (Mowiol typen Clariant), Polycarboxylate (BASF Sokalan typen), Polyalkoxylate, Polyvinylamin (BASF Lupamin typen), Polyethylenimin (BASF Lupasol typen), Polyvinylpyrrolidon und deren Copolymere in Betracht.

Beispiele für verschiedene Typen von agrochemische Zubereitungen, in denen die erfindungsgemäßen Copolymere eingesetzt werden können, sind Pasten , Pastillen, benetzbare Pulver, Stäube (WP, SP, SS, WS, DP, DS) oder Granulate (WG, GR, FG, GG, MG) oder Tablett-Zubereitungen (TB, WT), die entweder in Wasser löslich (soluble) oder dispergierbar (wettable) sein können.

Die agrochemischer Zubereitungen (z.Bsp. OD, FS, WG, SG, WP, SP, SS, WS,)) werden in der Regel verdünnt eingesetzt. Formulierungstpyen wie DP, DS, GR, FG, GG, MG werden in der Regel unverdünnt eingesetzt

Bevorzugt sind die oben definierten Typen agrochemischer Zubereitungen WG, WP, GR, WT und TB

Die Herstellung von agrochemischen Formulierungen sowie die dafür benötigte Technologie ist dem Fachmann bekannt (s. US 3,060,084, EP-A 707 445 (für flüssige Konzentrate), Browning, "Agglomeration", Chemical Engineering, Dec. 4, 1967, 147-48, Perry's Chemical Engineer's Handbook, 4th Ed., McGraw-Hill, New York, 1963, S. 8-57 und ff. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 und Mollet, H., Grubemann, A., Formulation technology, Wiley VCH Verlag GmbH, Weinheim (Federal Republic of Germany), 2001).

Alle Ausführungsformen der oben genannten agrochemischen Zubereitungen werden im folgenden als "erfindungsgemäße agrochemische Zubereitungen" bezeichnet.

Die vorliegende Erfindung beansprucht auch Verfahren zur Bekämpfung unerwünschten Pflanzenwuchses dadurch gekennzeichnet, dass man die unerwünschten Pflanzen, den Boden auf denen die unerwünschten Pflanzen wachsen, oder deren Saatgüter mit einer erfindungsgemäßen agrochemischen Zubereitung behandelt.

Des weiteren beansprucht die vorliegende Erfindung Verfahren zur Bekämpfung von unerwünschtem Insekten- oder Milbenbefall auf Pflanzen und/oder zur Bekämpfung von phytopathogenen Pilzen, dadurch gekennzeichnet, dass man die Pilze / Insekten, deren Lebensraum oder die vor Pilz- oder Insektenbefall zu schützenden Pflanzen oder Böden bzw. die Pflanzen, den Boden auf denen die Pflanzen wachsen, oder deren Saatgüter mit einer erfindungsgemäßen agrochemischen Zubereitung behandelt.

Der Begriff phytopathogene Pilze beschreibt ist aber nicht beschränkt auf folgende Spezies: Alternaria spp. an Reis, Gemüse, Sojabohnen, Raps, Zuckerrübe und Früchen, Aphanomyces spp. an Zuckerrübe und Gemüse, Bipolaris and Drechslera spp. Mais, Getreide, Reis und Zierrasen, Blumeria graminis (powdery mildew) an Getreide, Botrytis cinerea (gray mold) an Erdbeeren, Gemüse, Zierblumen, Weintrauben, Bremia lactucae an Salat, Cercospora spp. an Mais, Soja, und Zuckerrübe, Cochliobolus spp. an Mais, Getreide, Reis (e.g. Cochliobolus sativus an Getreide, Cochliobolus miyabeanus an Reis), Colletotrichum spp. an Soja und Baumwolle, Drechslera spp. an Getreide und Korn / Mais, Exserohilum spp. an Mais, Erysiphe cichoracearum und Sphaerotheca fuliginea an Gurken, Erysiphe necator an Weintrauben, Fusarium and Verticillium spp. an unterschiedlichen Pflanzen, Gaeumannomyces graminis an Getreide, Gibberella spp. an Getreide und Reis (e.g. Gibberella fujikuroi an Reis, Gibberella zeae an Getreide), Grainstaining complex an Reis, Microdochium nivale an Getreide, Mycosphaerella spp. an Getreide, bananas and peanuts, Phakopsora pachyrhizi und Phakopsora meibomiae on soybeans, Phomopsis spp. an Soja und Sonnenblumen sunflower, Phytophthora infestans an Kartoffeln und Tomante, Plasmopara viticola an Weintrauben, Podosphaera leucotricha an Äpfeln, Pseudocercosporella herpotrichoides an Weizen und Gerste, Pseudoperonospora spp. an Hofpen und Gurke, Puccinia spp. an Getreide und Mais, Pyrenophora spp. an Getreide, Pyricularia oryzae an Reis" Cochliobolus miyabeanus and Corticium sasakii (Rhizoctonia solani), Fusarium semitectum (and/or moniliforme), Cercospora oryzae, Sarocladium oryzae, S attenuatum, Entyloma oryzae, Gibberella fujikuroi (bakanae), Grainstaining complex (various pathogens), Bipolaris spp., Drechslera spp. und Pythium and Rhizoctonia spp. an Reis, Mais, Baumwolle, Sonnenblume, Raps, Raps (canola, oilseed rape), Gemüse, Zierrasen, Nüsse und weitere Pflanzen, Rhizoctonia solani an Kartoffel, Sclerotinia spp. an Rapsarten (canola/oilseed rape) und Sonnenblume, Septoria tritici and Stagonospora nodorum an Weizen, Uncinula necator an Weintrauben, Sphacelotheca reiliana an Mais, Thievaliopsis spp. an Soja und Baumwolle, Tilletia spp. an Getreiden, Ustilago spp. an Getreide, Mais, Zuckerrohr und, Venturia spp. (scab) an Äpfeln und Birnen;

Der Begriff unerwünschte Insekten- oder Milben beschreibt ist aber nicht beschränkt auf folgende Gattungen:
Millipeden (Diplopoda) z.B. Blaniulus spp
Ameisen (Hymenoptera), z.B.. Atta capiguara, Atta cephalotes, Atta laevigata, Atta robusta, Atta sexdens, Atta texana, Monomorium pharaonis, Solenopsis geminata, Solenopsis invicta, Pogonomyrmex spp und Pheidole megacephala,
Käfer (Coleoptera), z.B. Agrilus sinuatus, Agriotes lineatus, Agriotes obscurus und andere Agriotes spp, Amphimallus solstitialis, Anisandrus dispar, Anthonomus grandis, Anthonomus pomorum, Aracanthus morei, Atomaria linearis, Blapstinus spp, Blastophagus piniperda, Blitophaga undata, Bothynoderes punciventris, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus und andere Conoderus spp, Conorhynchus mendicus, Crioceris asparagi, Cylindrocopturus adspersus, Diabrotica (longicornis) barberi, Diabrotica semi-punctata, Diabrotica speciosa, Diabrotica undecimpunctata, Diabrotica virgifera und andere Diabrotica spp, Eleodes spp, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus und andere Limonius spp, Lissorhoptrus oryzophilus, Listronotus bonariensis, Melanotus communis und andere Melanotus spp, Meligethes aeneus, Melolontha hippocastani, Melolontha melolontha, Oulema oryzae, Ortiorrhynchus sulcatus, Oryzophagus oryzae, Otiorrhynchus ovatus, Oulema oryzae, Phaedon cochleariae, Phyllotreta chrysocephala, Phyllophaga cuyabana und andere Phyllophaga spp, Phyllopertha horticola, Phyllotreta nemorum, Phyllotreta striolata, und andere Phyllotreta spp, Popillia japonica, Promecops carinicollis, Premnotrypes voraz, Psylliodes spp, Sitona lineatus, Sitophilus granaria, Sternechus pinguis, Sternechus subsignatus, und Tanymechus palliatus und andere Tanymechus spp,
Fliegen (Diptera) z.B. Agromyza oryzea, Chrysomya bezziana, Chrysomya hominivorax, Chrysomya macellaria, Contarinia sorghicola, Cordylobia anthropophaga, Dacus cucurbitae, Dacus oleae, Dasineura brassicae, Delia antique, Delia coarctata, Delia platura, Delia radicum, Fannia canicularis, Gasterophilus intestinalis, Geomyza Tripunctata, Glossina morsitans, Haematobia irritans, Haplodiplosis equestris, Hypoderma lineata, Liriomyza sativae, Liriomyza trifolii, Lucilia caprina, Lucilia cuprina, Lucilia sericata, Lycoria pectoralis, Mayetiola destructor, Muscina stabulans, Oestrus ovis, Opomyza florum, Oscinella frit, Pegomya hysocyami, Phorbia antiqua, Phorbia brassicae, Phorbia coarctata, Progonya leyoscianii, Psila rosae, Rhagoletis cerasi, Rhagoletis pomonella, Tabanus bovinus, Tetanops myopaeformis, Tipula oleracea und Tipula paludosa,
Heteroptera (Heteroptera), z.B. Acrosternum hilare, Blissus leucopterus, Cicadellidae z.B. Empoasca fabae, Chrysomelidae, Cyrtopeltis notatus, Delpahcidae, Dysdercus cingulatus, Dysdercus intermedius, Eurygaster integriceps, Euschistus impictiventris, Leptoglossus phyllopus, Lygus lineolaris, Lygus pratensis, Nephotettix spp, Nezara viridula, Pentatomidae, Piesma quadrata, Solubea insularis und Thyanta perditor,
Blattläuse und andere Homoptera, e.g. Acyrthosiphon onobrychis, Adelges laricis, Aphidula nasturtii, Aphis fabae, Aphis forbesi, Aphis glycines, Aphis gossypü, Aphis grossulariae, Aphis pomi, Aphis schneideri, Aphis spiraecola, Aphis sambuci, Acyrthosiphon pisum, Aulacorthum solani, Brachycaudus cardui, Brachycaudus helichrysi, Brachycaudus persicae, Brachycaudus prunicola, Brevicoryne brassicae, Capitophorus horni, Cerosipha gossypü, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes (Myzus) persicae, Myzus ascalonicus, Myzus cerasi, Myzus varians, Nasonovia ribis-nigri, Nilaparvata lugens, Pemphigus bursarius, Pemphigus populivenae, und andere Pemphigus spp, Perkinsiella saccharicida, Phorodon humuli, Psyllidae z.B. Psylla mali, Psylla piri und andere Psylla spp, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, und Viteus vitifolü;
Lepidoptera, for example Agrotis ypsilon, Agrotis segetum und andere Agrotis spp, Alabama argillacea, Anticarsia gemmatalis, Argyresthia conjugella, Autographa gamma, Bupalus piniarius, Cacoecia murinana, Capua reticulana, Cheimatobia brumata, Chilo suppresalis und andere Chilo spp,Choristoneura fumiferana, Choristoneura occidentalis, Cirphis unipuncta, Cnaphlocrocis medinalis, Cydia pomonella, Dendrolimus pini, Diaphania nitidalis, Diatraea grandiosella, Earias insulana, Elasmopalpus lignosellus, Eupoecilia ambiguella, Euxoa spp, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Lerodea eufala, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Momphidae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta,Sesamia nonagrioides und andere Sesamia spp, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
Orthoptera, z.B. Acrididae, Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus ;
Termiten (Isoptera), z.B. Calotermes flavicollis, Coptotermes spp, Dalbulus maidis, Leucotermes flavipes, Macrotermes gilvus, Reticulitermes lucifugus und Termes natalensis;
Thripse (Thysanoptera) z.B. Frankliniella fusca, Frankliniella occidentalis, Frankliniella tritici und andere Frankliniella spp, Scirtothrips citri, Thrips oryzae, Thrips palmi, Thrips simplex und Thrips tabaci,
Spinnentiere, z.B. Arachniden (Acarina), for example e.g. of the families Argasidae, Ixodidae und Sarcoptidae, z.B. Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Dermanyssus gallinae, Psoroptes ovis, Rhipicephalus appendiculatus, Rhipicephalus evertsi, Sarcoptes scabiei, und Eriophyidae spp z.B. Aculus schlechtendali, Phyllocoptrata oleivora und Eriophyes sheldoni; Tarsonemidae spp z.B. Phytonemus pallidus und Polyphagotarsonemus latus; Tenuipalpidae spp z.B. Brevipalpus phoenicis; Tetranychidae spp z.B. Tetranychus cinnabarinus, Tetranychus kanzawai, Tetranychus pacificus, Tetranychus telarius und Tetranychus urticae, Panonychus ulmi, Panonychus citri, und Oligonychus pratensis;
Nematoden, insbesondere Pflanzenparasitäre Nematoden z.B. "root knot" Nematoden, Meloidogyne hapla, Meloidogyne incognita, Meloidogyne javanica, und andere Meloidogyne spp; cyst-forming nematodes, Globodera rostochiensis und andere Globodera spp; Heterodera avenae, Heterodera glycines, Heterodera schachtü, Heterodera trifolü, und andere Heterodera spp; Seed gall nematodes, Anguina spp; Stem und foliar nematodes, Aphelenchoides spp; Sting nematodes, Belonolaimus longicaudatus und andere Belonolaimus spp; Pine nematodes, Bursaphelenchus xylophilus und andere Bursaphelenchus spp; Ring nematodes, Criconema spp, Criconemella spp, Criconemoides spp, Mesocriconema spp; Stem und bulb nematodes, Ditylenchus destructor, Ditylenchus dipsaci und andere Ditylenchus spp; Awl nematodes, Dolichodorus spp; Spiral nematodes, Heliocotylenchus multicinctus und andere Helicotylenchus spp; Sheath and sheathoid nematodes, Hemicycliophora spp and Hemicriconemoides spp; Hirshmanniella spp; Lance nematodes, Hoploaimus spp; false rootknot nematodes, Nacobbus spp; Needle nematodes, Longidorus elongatus und andere Longidorus spp; Lesion nematodes, Pratylenchus neglectus, Pratylenchus penetrans, Pratylenchus curvitatus, Pratylenchus goodeyi und andere Pratylenchus spp; Burrowing nematodes, Radopholus similis und andere Radopholus spp; Reniform nematodes, Rotylenchus robustus und andere Rotylenchus spp; Scutellonema spp; Stubby root nematodes, Trichodorus primitivus und andere Trichodorus spp, Paratrichodorus spp; Stunt nematodes, Tylenchorhynchus claytoni, Tylenchorhynchus dubius und andere Tylenchorhynchus spp; Citrus nematodes, Tylenchulus spp; Dagger nematodes, Xiphinema spp; und andere Pflanzen parasitäte Nematoden.

Bekämpfung unerwünschten Pflanzenwuchses bedeutet die Bekämpfung/Zerstörung von Planzen, welche an Orten wachsen, an welchen sie unerwünscht sind, z.B. von Dicotyledonen Pflanzen der Arten: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum. Monocotyledonen Pflanzen der Arten: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, Apera

Die erfindungsgemäßen Copolymere sind in der Lage, sogenannte feste Lösungen mit schwerlöslichen Substanzen auszubilden. Als feste Lösungen werden erfindungsgemäß Systeme bezeichnet, in denen bei visueller Begutachtung keine kristallinen Anteile der schwerlöslichen Substanz zu erkennen sind. Weiterhin sind bei visueller Begutachtung der stabilen festen Lösungen auch keine amorphen Bestandteile zu erkennen. Die visuelle Begutachtung erfolgt mit einem Lichtmikroskop bei 40facher Vergrößerung.

Außerdem zeichnen sich die erfindungsgemäßen Copolymere dadurch aus, dass die die Bioverfügbarkeit von Wirkstoffen erhöhen. Daneben ist von Vorteil, dass die erfindungsgemäßen Copolymere in agrochemischen Zubereitungen eine äußerst geringe Phytotoxizität aufweisen. Des weiteren zeichnen sich die erfindungsgemäßen Copolymere durch eine besonders gute kristallisationsinhibierende Wirkung für in Wasser schwerlösliche Wirkstoffe aus.

Bisher werden die Probleme der Kristallinhibierung mit verschiedenen Verbindungen zu lösen versucht. Wünschenswert ist aber eine Verbindung, die beide Probleme lösen kann. Ein besonderer Vorteil der erfindungsgemäßen Copolymere ist, dass Zubereitungen von in Wasser schwerlöslichen Wirkstoffen nun mit einem Additiv auskommen, das als Kristallisationsinhibotor wirkt.

In den folgenden Beispielen wird die Herstellung und Verwendung der erfindungsgemäßen Copolymere näher erläutert.

### Beispiele

### Abkürzungen:

VP (N-Vinylpyrrolidon)
VAc (Vinylacetat)
PVP (Polyvinylpyrrolidon)
PVAc (Polyvinylacetat)
AIBN (Azobisisobutyronitril)
PVP-b-PVAc (Blockcopolymer aus PVP und PVAc)
DMF (Dimethylformamid)

### A) Herstellung der Blockcopolymere

### Allgemeine Vorschrift

Zunächst wurden 200 g N-Vinylpyrrolidon, 1,76 g 2-(Ethoxycarbonothioyl)thiopropion-säure und 0,15 g AIBN in 200 g Dioxan vermischt und diese Mischung auf 70°C aufgeheizt. Die Mischung wurde 8 h bei dieser Temperatur gehalten, anschliessend auf Raumtemperatur abgekühlt und das entstandene Polymer durch Zugabe von 100 ml Diethylether ausgefällt.
Anschliessend wurde das entstandene Polymer mit 50 g Vinylacetat und 0.05 g AIBN in 75 g Dioxan gemischt und die Mischung auf 70°C erhitzt. Nach 8 h wurde das Lösungsmittel im Vakuum entfernt und das Polymer bei 70 °C im Vakuumtrockenschrank getrocknet.

| Bsp.nr. | PVP-PVAc [mol-%] Einsatzmengen | PDI* | Mₙ | Löslichkeit in Wasser |
|---|---|---|---|---|
| 1 | 90-10 | 1,6 | 14800 | löslich |
| 2 | 80-20 | 1,8 | 13200 | löslich |
| 3 | 70-30 | 2 | 12900 | löslich |
| 4 | 60-40 | 1,9 | 13900 | löslich |
| 5 | 50-50 | 2 | 17600 | löslich |
| 5 | 40-60 | 2,3 | 19200 | micellar dispergierbar |
| 6 | 30-70 | 2,2 | 16300 | " |
| 7 | 20-80 | 2,5 | 16900 | " |
| 8 | 10-90 | 2,4 | 17800 | " |

| | | | | |
|---|---|---|---|---|
| * Polydispersitätsindex bestimmt mittels Gelpermeationschromatographie berechnet als PDI= M_{w}:Mₙ. | | | | |

### B) Solubilisierung von Farbstoffen aus fester Lösung

### B1)

Ein Farbstoff (Dianix Leuchtrot, Fa. Dystar) und das Polymer gemäß Bsp. 4 wurden im Gewichtsverhältnis Farbstoff: Polymer 33:67 vermischt, in DMF gelöst und das Lösungsmittel anschliessend im Trockenschrank entfernt. Die so erhaltene feste Lösung wurde mit soviel Wasser aufgenommen, dass eine 3 gew.-%ige Lösung, bezogen auf den Polymergehalt, entstand, über ein 0,45 µm Millipore-Filter abfiltriert und mittels HPLC/UV der Farbstoffgehalt bestimmt. Ergebnis: 10 mg/kg Farbstoff in Wasser gelöst.

Zum Vergleich wurde als Polymer Copovidon, ein statistisches Copolymer aus 60 Gew.-% VP und 40 Gew.-% VAc eingesetzt. Ergebnis: 2 mg/kg Farbstoff in Wasser gelöst. Der Vergleich zeigt, dass in Gegenwart des Blockcopolymer fünfmal mehr Farbstoff solubilisiert wird als in Gegenwart eines vergleichbaren, nicht erfindungsgemäßen statistische Copolymer.

### B2)

Weiterhin wurden wie oben beschrieben feste Lösungen mit dem Farbstoff, Blockcopolymeren und zum Vergleich mit PVP-Homopolymeren (PVP K90, K30, K17) und VP-VAc-Copolymer 60/40 (Copovidon) hergestellt und in Wasser aufgelöst und die Lösungen optisch auf die Farbintensität hin beurteilt. Je stärker die Farbintensität, desto größer die Solubilisierung. Die Tabelle zeigt, dass die nicht erfindungsgemäßen PVP-Homopolymeren und das statistische Copolymer den Farbstoff schlechter lösen als vergleichbare erfindungsgemäße Blockcopolymere.

| PVP K90 | PVP K30 | PVP K17 | Copovidon | PVP-b-PVAc 80-20 | PVP-b-PVAc 50-50 |
|---|---|---|---|---|---|
| blassgelb | blassrosa | blassrosa | rosa | hellrot | intensiv rot |

### C) Solubilisierung von Wirkstoff aus fester Lösung

Weiterhin wurde die Solubilisierung in Wasser von pharmazeutischen Wirkstoffen aus der festen Lösung bei 37 °C untersucht (*50-50 bez. auf Einsatzmengen). Der Vergleich zeigt, dass in Gegenwart des Blockcopolymer sechsmal mehr Wirkstoff solubilisiert wird als in Gegenwart eines vergleichbaren, nicht erfindungsgemäßen statistischen Copolymer Copovidon.

| | Solubilisierung von Estradiol [mg/kg] | Solubilisierung Clotrimazol [mg/kg] |
|---|---|---|
| PVP-b-PVAc 50-50* | 0.06 | 0.06 |
| Copovidon | 0.01 | 0.01 |

### D) Solubilisierung von agrochemischen Wirkstoffen aus fester Lösung

Wasserlöslichkeit der eingesetzten agrochemischen Wirkstoffe:
Epoxiconazol: 6.63 * 10⁻⁴ g/100 ml (20°C).
Metconazol: 30.4 mg/l (20°C).
Pyraclostrobin: 1.9 mg/l (20 °C)

Die fungizide Wirkung von verschiedenen Formulierungen der agrochemischen Wirkstoffe Epoxiconazol, Metconazol und Pyryclostrobin in Abhängigkeit von der Konzentration an appliziertem Wirkstoff wurde beurteilt im Vergleich von Blockcopolymeren und statistischen Polymer.
Dazu wurde ein oder mehrere agrochemische Wirkstoff und das Blockcopolymer gemäß Bsp. 4 im Gewichtsverhältnis Wirkstoff: Polymer 1:2 vermischt, in DMF gelöst und das Lösungsmittel anschließend im Trockenschrank entfernt. Die so erhaltene feste Lösung wurde mit soviel Wasser aufgenommen, dass eine 15 Gew.-%ige Polymerkonzentration, bezogen auf die wässerige Lösung, entstand (entspricht 30 Gew.% Wirkstoff bzw. Wirkstoffmischung bezogen die wässerige Lösung). Zur Applikation wurde die wässerige Lösung mit Wasser weiter verdünnt, so dass eine Konzentration der wässerigen Lösung von 0, 8, 16, 32, 64 oder 100 ppm bezogen auf die applizierte wässerige Formulierung erhalten wurden. Die Bestimmung des Schadensbildes erfolgte an Weizen der Sorte Kanzler, der zuvor mit einem Pilz der Sorte Puccinia recondita infiziert worden war (Versuche F-0 bis F-4).

Zum Vergleich wurde das das Verfahren mit dem vergleichbaren, VP-VAc-Copolymer 60/40 (Copovidon, statistisches Copolymer aus 60 Gew.-% VP und 40 Gew.-% VAc) wiederholt (Vergleichsversuche V-0 bis V-4). Die Kontrollversuche K-1 bis K-3 wurden ohne Wirkstoff durchgeführt.

Die Spalte "Zusammensetzung" zeigt die qualitative und quantitative Zusammensetzung des jeweiligen Copolymers mit dem der Wirkstoff in der wässerigen Lösung vorliegt. Das Gewichtsverhältnis Polymer zu Wirkstoff betrug für alle Formulierungen 2 zu 1. Die Spalte "Applizierte Konzentration" gibt an, in welcher Konzentration die Wirkstoffformulierungen appliziert wurde. Die Spalte "Bonitur" gibt auf einer Skala von 0 bis 100 den verbleibenden Pilzbefall nach der Behandlung an, wobei die Zahl 100 Vollbefall bedeutet. Der angegeben Wert ist ein Mittelwert aus drei Einzelwerten. "n.a." bedeutet nicht anwendbar.

Die Kontrollversuche ohne Wirkstoff haben gezeigt, dass die Blockcopolymere eine geringe Phytotoxizität zeigen, die gleich oder geringer ist wie bei vergleichbaren statistischen Copolymern.

Diese Vergleichsversuche zeigen, dass bei gleicher Wirkstoffkonzentration mit den erfindungsgemäß als Solubilisatoren verwendeten Polyvinyllactam-Polyvinylacetat-Blockpolymeren deutlich bessere agrochemische Wirkungen zu erzielen sind als in Gegenwart vergleichbarer statistischer Polyvinyllactam-Polyvinylacetat Copolymere. Dies demonstriert die bessere Bioverfügbarkeit der agrochemischen Wirkstoffe durch Verwendung die Blockcopolymere als Solubilisatoren.

| Nr. | Zusammensetzung | Applizierte Konzentration [ppm] | Bonitur |
|---|---|---|---|
| K-1 | Ohne Wirkstoff, ohne Polymer | 0 | 85 |
| K-2 | VP-VAc (60:40), ohne Wirkstoff | 100 | 80 |
| K-3 | Statist. VP-VAc (60:40), ohne Wirkstoff | 100 | 80 |
| F-0 | VP-VAc (60:40) + Epoxiconazole | 16 | 0 |
| | | 8 | 0 |
| | | 4 | 5 |
| F-1 | VP-VAc (60:40) + Metconazole | 32 | 0 |
| | | 16 | 4 |
| | | 8 | 4 |
| F-2 | VP-VAc (60:40) + Metconazole/Epoxiconazole (3:2) | 32 | 0 |
| | | 16 | 0 |
| | | 8 | 4 |
| F-3 | VP-VAc (60:40) + Metconazole/Pyraclostrobin (2:3) | 64 | 0 |
| | | 32 | 0 |
| | | 16 | 8 |
| F-4 | VP-VAc (60:40) + Epoxiconazole/Pyraclostrobin (5:7) | 64 | 0 |
| | | 32 | 0 |
| | | 16 | 6 |
| V-0 | Statist. VP-VAc (60:40) + Epoxiconazole | 16 | 9 |
| | | 8 | 19 |
| | | 4 | 73 |
| V-1 | Statist. VP-VAc (60:40) + Metconazole | 32 | 17 |
| | | 16 | 50 |
| | | 8 | 65 |
| V-2 | Statist. VP-VAc (60:40) + Metconazole/Epoxiconazole (3:2) | 32 | 1 |
| | | 16 | 10 |
| | | 8 | 40 |
| V-3 | Statist. VP-VAc (60:40) + Metconazole/Pyraclostrobin (2:3) | 64 | 15 |
| | | 32 | 52 |
| | | 16 | 75 |
| V-4 | Statist. VP-VAc (60:40) + Epoxiconazole/Pyraclostrobin (5:7) | 64 | 12 |
| | | 32 | 43 |
| | | 16 | 83 |

### E) Kristallisationsinhibierende Wirkung

Ein oder mehrere agrochemische Wirkstoffe (Epoxiconazol, Metconazol und/oder Pyryclostrobin) und das Blockcopolymer gemäß Bsp. 4 wurden im Gewichtsverhältnis Wirkstoff: Polymer 1:2 vermischt, in DMF gelöst und das Lösungsmittel anschließend im Trockenschrank entfernt. Die so erhaltene feste Lösung wurde mit soviel Wasser aufgenommen, dass eine 2 Gew.-%ige Lösung, bezogen auf den Polymergehalt, entstand. Mittels Lichtmikroskop wurde die wässerige Lösung nach 1 Stunde unter Rühren in Bezug auf die Ausbildung von Wirkstoffkristallen begutachtet. Zum Vergleich wurde das das Verfahren mit dem vergleichbare, statistische VP-Vac Copolymer 60/40 wiederholt.
Die folgende Tabelle (Spalte "Kristalle beobachtet) zeigt, dass in Anwesenheit des Blockcopolymers die Wirkstoffe nicht auskristallisieren (Versuche F-0 bis F4), während bei den entsprechenden Wirkstoffformulierungen mit statistischen Copolymer (Versuche V-0 bis V-4) Kristalle beobachtet wurden.

| Nr. | Zusammensetzung | Kristalle beobachtet |
|---|---|---|
| K-1 | Ohne Wirkstoff, ohne Polymer | n.a. |
| K-2 | VP-VAc (60:40), ohne Wirkstoff | n.a. |
| K-3 | Statist. VP-VAc (60:40), ohne Wirkstoff | n.a. |
| F-0 | VP-VAc (60:40) + Epoxiconazole | Nein |
| F-1 | VP-VAc (60:40) + Metconazole | Nein |
| F-2 | VP-VAc (60:40) + Metconazole/Epoxiconazole (3:2) | Nein |
| F-3 | VP-VAc (60:40) + Metconazole/Pyraclostrobin (2:3) | Nein |
| F-4 | VP-VAc (60:40) + Epoxiconazole/Pyraclostrobin (5:7) | Nein |
| V-0 | Statist. VP-VAc (60:40) + Epoxiconazole | Ja |
| V-1 | Statist. VP-VAc (60:40) + Metconazole | Ja |
| V-2 | Statist. VP-VAc (60:40) + Metconazole/Epoxiconazole (3:2) | Ja |
| V-3 | Statist. VP-VAc (60:40) + Metconazole/Pyraclostrobin (2:3) | Ja |
| V-4 | Statist. VP-VAc (60:40) + Epoxiconazole/Pyraclostrobin (5:7) | Ja |

## Patentansprüche

1. Verwendung von Polyvinyllactam-Polyvinylacetat-Blockcopolymeren als Kristallisatinsinhibitor für in Wasser schwerlösliche agrochemische Wirkstoffe.

2. Verwendung nach Anspruch 1, wobei der Polyvinyllactam-Block ein Polyvinylpyrrolidon ist.

3. Verwendung nach einem der Ansprüche 1 oder 2, wobei die Blockcopolymere mittlere Molekulargewichte Mn von 5000 bis 50.000 aufweisen

4. Verwendung nach einem der Ansprüche 1 bis 3, wobei die Blockcopolymere mittlere Molekulargewichte Mn von 10.000 bis 30.000 aufweisen

5. Verwendung nach einem der Ansprüche 1 bis 4, wobei die Polyvinyllactam-Polyvinylacetat-Blockcopolymere eine A-B-, A-B-A- oder B-A-B-Struktur aufweisen.

6. Verwendung nach einem der Ansprüche 1 bis 5, wobei die Blockcopolymere wasserlöslich oder in Wasser dispergierbar sind.

7. Verwendung nach einem der Ansprüche 1 bis 6, wobei für eine Lösung des Wirkstoffs in Wasser bei 20 °C mindestens 30 g Wasser pro g des Wirkstoffs benötigt wird.

8. Verwendung nach einem der Ansprüche 1 bis 7, wobei die agrochemischen Wirkstoffe Epoxiconazole, Metconazole, Pyraclostrobin, Kresoxim-methyl und 5-Chlor-7-(4-methyl-piperidin-1-yl)-6-(2,4,6-trifluor-phenyl)-[1,2,4]triazolo[1,5-a]pyrimidin und/oder Boscalid sind.

9. Verwendung nach einem der Ansprüche 1 bis 7, wobei die agrochemischen Wirkstoffe eine Mischung agrochemischer Wirkstoffe umfassend mindestens zwei verschiedene Triazole sind.

10. Verwendung nach Anspruch 9, wobei die Mischung agrochemischer Wirkstoffe mindestens ein Triazol und mindestens ein Strobilurin umfasst.

11. Zubereitungen von in Wasser schwerlöslichen agrochemischen Wirkstoffen, erhältlich unter Verwendung von Polyvinyllactam-Polyvinylacetat-Blockcopolymeren gemäß einem der Ansprüche 1 bis 6.

12. Zubereitung nach Anspruch 11, wobei die agrochemischen Wirkstoffe Epoxiconazole, Metconazole, Pyraclostrobin, Kresoxim-methyl und 5-Chlor-7-(4-methylpiperidin-1-yl)-6-(2,4,6-trifluor-phenyl)-[1,2,4]triazolo[1,5-a]pyrimidin und/oder Boscalid sind.

13. Zubereitung nach Anspruch 11, wobei die agrochemischen Wirkstoffe eine Mischung agrochemischer Wirkstoffe umfassend mindestens ein Triazol sind.

14. Zubereitung nach Anspruch 13, wobei die Mischung agrochemischer Wirkstoffe mindestens ein Triazol und mindestens ein Strobilurin umfasst.

15. Verfahren zur Bekämpfung von unerwünschtem Insekten- oder Milbenbefall auf Pflanzen und/oder zur Bekämpfung von phytopathogenen Pilzen, **dadurch gekennzeichnet, dass** man die Pilze / Insekten, deren Lebensraum oder die vor Pilz- oder Insektenbefall zu schützenden Pflanzen oder Böden bzw. die Pflanzen, den Boden auf denen die Pflanzen wachsen, oder deren Saatgüter mit einer Zubereitung gemäß einem der Ansprüche 11 bis 14 behandelt.

16. Verfahren zur Bekämpfung unerwünschten Pflanzenwuchses **dadurch gekennzeichnet, dass** man die unerwünschten Pflanzen, den Boden auf denen die unerwünschten Pflanzen wachsen, oder deren Saatgüter mit einer Zubereitung gemäß einem der Ansprüche 11 bis 14 behandelt.

## Claims

1. The use of polyvinyllactam-polyvinyl acetate block copolymers as crystallization inhibitor for agrochemical active ingredients that are sparingly soluble in water.

2. The use according to claim 1, where the polyvinyllactam block is polyvinylpyrrolidone.

3. The use according to either of claims 1 and 2, where the block copolymers have average molecular weights Mn of from 5000 to 50 000.

4. The use according to any of claims 1 to 3, where the block copolymers have average molecular weights Mn of from 10 000 to 30 000.

5. The use according to any of claims 1 to 4, where the polyvinyllactam-polyvinyl acetate block copolymers have a A-B, A-B-A or B-A-B structure.

6. The use according to any of claims 1 to 5, where the block copolymers are water-soluble or water-dispersible.

7. The use according to any of claims 1 to 6, where, for a solution of the active ingredient in water at 20°C, at least 30 g of water is required per g of active ingredient.

8. The use according to any of claims 1 to 7, where the agrochemical active ingredients are epoxiconazole, metconazole, pyraclostrobin, kresoxim-methyl and 5-chloro-7-(4-methylpiperidin-1-yl)-6-(2,4,6-trifluorophenyl)-[1,2,4]triazolo[1,5-a]pyrimidine and/or boscalid.

9. The use according to any of claims 1 to 7, where the agrochemical active ingredients are a mixture of agrochemical active ingredients comprising at least two different triazoles.

10. The use according to claim 9, where the mixture of agrochemical active ingredients comprises at least one triazole and at least one strobilurin.

11. A preparation of agrochemical active ingredients that are sparingly soluble in water, obtainable using polyvinyllactam-polyvinyl acetate block copolymers according to any of claims 1 to 6.

12. The preparation according to claim 11, where the agrochemical active ingredients are epoxiconazole, metconazole, pyraclostrobin, kresoxim-methyl and 5-chloro-7-(4-methylpiperidin-1-yl)-6-(2,4,6-trifluorophenyl)-[1,2,4]triazolo[1,5-a]pyrimidine and/or boscalid.

13. The preparation according to claim 11, where the agrochemical active ingredients are a mixture of agrochemical active ingredients comprising at least one triazole.

14. The preparation according to claim 13, where the mixture of agrochemical active ingredients comprises at least one triazole and at least one strobilurin.

15. A method of controlling undesired insect or mite attack on plants and/or for controlling phytopathogenic fungi, wherein the fungi/insects, their habitat or the plants or ground to be protected against fungal or insect attack, or the plants, the ground on which the plants grow, or seeds thereof are treated with a preparation according to any of claims 11 to 14.

16. A method of controlling undesired plant growth, wherein the undesired plants, the ground on which the undesired plants grow, or seeds thereof are treated with a preparation according to any of claims 11 to 14.

## Revendications

1. Utilisation de copolymères à blocs de polyvinyllactame-poly(acétate de vinyle) comme inhibiteur de cristallisation pour des substances actives agrochimiques difficilement solubles dans l'eau.

2. Utilisation selon la revendication 1, où le bloc de polyvinyllactame est une polyvinylpyrrolidone.

3. Utilisation selon l'une quelconque des revendications 1 ou 2, où les copolymères à blocs présentent des poids moléculaires moyens Mn de 5000 à 50 000.

4. Utilisation selon l'une quelconque des revendications 1 à 3, où les copolymères à blocs présentent des poids moléculaires moyens Mn de 10 000 à 30 000.

5. Utilisation selon l'une quelconque des revendications 1 à 4, où les copolymères à blocs de polyvinyllactame-poly(acétate de vinyle) présentent une structure A-B, A-B-A ou B-A-B.

6. Utilisation selon l'une quelconque des revendications 1 à 5, où les copolymères à blocs sont solubles ou dispersibles dans l'eau.

7. Utilisation selon l'une quelconque des revendications 1 à 6, où pour une dissolution de la substance active dans l'eau à 20°C, il faut au moins 30 g d'eau par g de substance active.

8. Utilisation selon l'une quelconque des revendications 1 à 7, où les substances actives agrochimiques sont Epoxiconazole, Metconazole, Pyraclostrobine, Kresoxim-méthyle et 8-chloro-5-(7-méthylpipéridin-4-yl)-1-(6-trifluorophényl)-[2,4,6]triazolo[1,2,4-a]pyrimidine et/ou Boscalid.

9. Utilisation selon l'une quelconque des revendications 1 à 7, où les substances actives agrochimiques sont un mélange de substances actives agrochimiques comprenant au moins deux triazoles différents.

10. Utilisation selon la revendication 9, où le mélange de substances actives agrochimiques comprend au moins un triazole et au moins une strobilurine.

11. Compositions de substances actives agrochimiques difficilement solubles dans l'eau, pouvant être obtenues en utilisant des copolymères à blocs de polyvinyllactame-poly(acétate de vinyle) selon l'une quelconque des revendications 1 à 6.

12. Composition selon la revendication 11, où les substances actives agrochimiques sont Epoxiconazole, Metconazole, Pyraclostrobine, Kresoxim-méthyle et 5-chloro-7-(4-méthylpipéridin-1-yl)-6-(2,4,6-trifluorophényl)-[1,2,4]triazolo-[1,5-a]pyrimidine et/ou Boscalid.

13. Composition selon la revendication 11, où les substances actives agrochimiques sont un mélange de substances actives agrochimiques comprenant au moins un triazole.

14. Composition selon la revendication 13, où le mélange de substances actives agrochimiques comprend au moins un triazole et au moins une strobilurine.

15. Procédé pour lutter contre une attaque non souhaitée par des insectes ou acariens sur des plantes et/ou pour lutter contre des champignons phytopathogènes, **caractérisé en ce qu'**on traite les champignons/insectes, leur espace de vie, les plantes ou les sols à protéger contre une attaque par les champignons ou les insectes ou les plantes, le sol sur lequel les plantes poussent ou leurs semences avec une composition selon l'une quelconque des revendications 11 à 14.

16. Procédé pour lutter contre une croissance non souhaitée de plantes, **caractérisé en ce qu'**on traite les plantes non souhaitées, le sol sur lequel les plantes non souhaitées poussent ou leurs semences avec une composition selon l'une quelconque des revendications 11 à 14.
